(19) Europäisches Patentamt
European Patent Office
Office européen des brevets

(11) Numéro de publication: **0 240 392 B1**

# (12) FASCICULE DE BREVET EUROPEEN

(45) Date de publication du fascicule du brevet: **09.05.90**

(51) Int. Cl.[5]: **C07F 9/547,** A61K 31/675

(21) Numéro de dépôt: **87400490.6**

(22) Date de dépôt: **05.03.87**

## (54) Composés du type cyclophosphazène, procédé de préparation et composition pharmaceutique les contenant.

(30) Priorité: **06.03.86 FR 8603155**

(43) Date de publication de la demande: **07.10.87 Bulletin 87/41**

(45) Mention de la délivrance du brevet: **09.05.90 Bulletin 90/19**

(84) Etats contractants désignés: **AT BE CH DE ES FR GB GR IT LI LU NL SE**

(56) Documents cités:
**EP-A- 0 112 743**

**INORGANICA CHIMICA ACTA, vol. 108, 1985, pages 23-27, Elsevier Sequoia; J.-L. SASSUS et al.: "Covalent binding of non-effective diaziridinocyclotriphosphazenes to natural polyamines as tumor finders makes potential anticancer agents"**

(73) Titulaire: **CENTRE NATIONAL DE LA RECHERCHE SCIENTIFIQUE (CNRS), 15, Quai Anatole France, F-75007 Paris(FR)**

(72) Inventeur: **Labarre, Jean-François, 30, rue des Géraniums, F-31400 Toulouse(FR)**
Inventeur: **Sournies, François, 5, rue du Moulin, F-31520 Ramonville Saint Agbe(FR)**

(74) Mandataire: **Warcoin, Jacques et al, Cabinet Régimbeau 26, avenue Kléber, F-75116 Paris(FR)**

EP 0 240 392 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés chimiques nouveaux, des procédés pour leur préparation et l'application de ces composés à titre de médicaments.

Les composés selon la présente invention sont apparentés aux dérivés hétérocycliques non carbonés de type cyclophosphazènes dont un certain nombre ont déjà été décrits dans la littérature.

Les cycles inorganiques antitumoraux de première génération (cyclophosphazènes du type MYKO 63) et de deuxième génération (cyclodiphosphathiazènes du type SoAz) se caractérisent d'une manière générale par une spécificité trop faible pour les cellules malignes, les effets thérapeutiques n'étant obtenus qu'avec des doses proches de la dose maximale non léthale (DLo), d'où apparition d'effets secondaires (essentiellement de thrombocytopénies) importants puisque dose-dépendants.

C'est pour remédier à ce défaut qu'il a été entrepris depuis 1981 d'augmenter la spécificité de ces drogues de première et de deuxième génération en les accrochant de manière covalente à des vecteurs présentant une affinité pour les tumeurs que constituent les polyamines biogènes, c'est-à-dire le 1,3-diaminopropane, la putrescine, la cadavérine, la spermidine, l'agmatine et la spermine.

Les bénéfices tirés d'une telle vectorisation par les polyamines accrochées à la drogue en configuration SPIRO ont été clairement démontrés et sont décrits dans le brevet français n° 82 19768 déposé le 25 novembre 1982 au nom du Centre National de la Recherche Scientifique (CNRS).

Une telle vectorisation conduit à une diminution par un facteur de 3 à 5 de la toxicité (DLo) de la drogue et surtout une augmentation considérable de l'index thérapeutique (IT) (défini comme le rapport de la DLo à la dose minimale active en mono-injection) qui passe de la valeur 4 pour la drogue «nue» à 10–15 pour la drogue vectorisée.

L'article dans Inorganica Chimica Acta, vol. 108, pages 23–27, Elsevier Sequoia, de J.L. Sassus et col., décrit des composés dérivés de cyclotriphosphazènes utilisables potentiellement comme agents anticancéreux.

La présente invention a plus particulièrement pour objet des super-systèmes dans lesquels une molécule de polyamine entraîne avec elle deux molécules de drogue.

Il s'agit de composés de formule I:

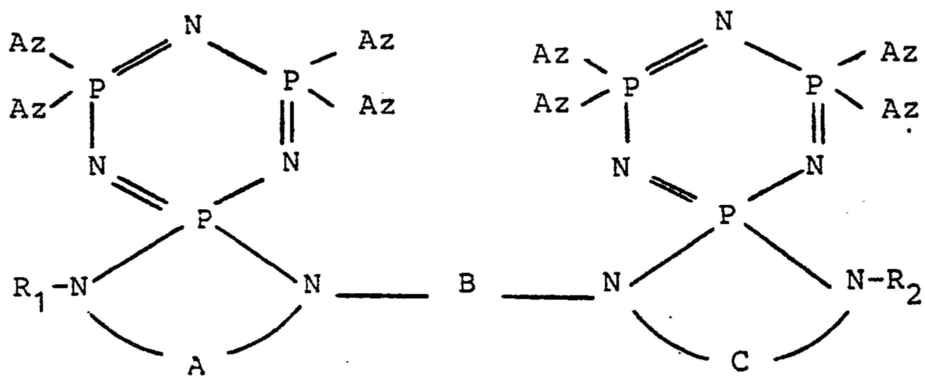

(I)

dans laquelle,

. Az est un radical 1-aziridinyle substitué ou non,

. A, B et C peuvent représenter,indépendamment l'un de l'autre, une chaîne alcoylène, alcénylène ou alcynylène, droite ou ramifiée, substituée ou non, qui peut être interrompue par un ou plusieurs radicaux divalents $-NR_3-$, -S- ou -O-,

. $R_1$, $R_2$ et $R_3$ peuvent représenter, indépendamment l'un de l'autre : un atome d'hydrogène, un radical alkyle substitué ou non, un radical alcényle substitué ou non, un radical alcynyle substitué ou non et un radical aryle substitué ou non.

Le radical 1-aziridinyle peut être substitué par 1 à 4 radicaux alkyles ou alcoxy en $C_1$ à $C_3$. Cependant, dans les composés de formule I, le radical Az est de préférence un radical 1-aziridinyle non substitué.

Les chaînes A, B et C sont, de préférence, des chaînes alcoylènes ayant 1 à 6 atomes de carbone. Elles peuvent être interrompues, de préférence par 1 à 4 radicaux du type $-NR_3-$.

Les radicaux $R_1$, $R_2$ et $R_3$ sont, de préférence, un atome d'hydrogène ou un radical alkyle, substitué ou non. Ces radicaux $R_1$, $R_2$ et $R_3$ peuvent, en outre, être substitués, comme les chaînes A, B et C, par un ou plusieurs atomes d'halogène, radicaux imino, amino, aryle non substitué ou substitué par un ou plusieurs atomes d'halogène ou radicaux alkyles en $C_1$ à $C_3$.

Par "radicaux alkyles, alcényles ou alcynyles", il faut entendre plus particulièrement les radicaux inférieurs, droits ou ramifiés, ayant de préférence de 1 à 7 atomes de carbone, notamment de 1 à 3 atomes de carbone.

Par "radical aryle", il faut entendre essentiellement des radicaux monocycliques, substitués ou non, tels que le radical phényle.

La présente invention concerne, en particulier, les composés de formule I' :

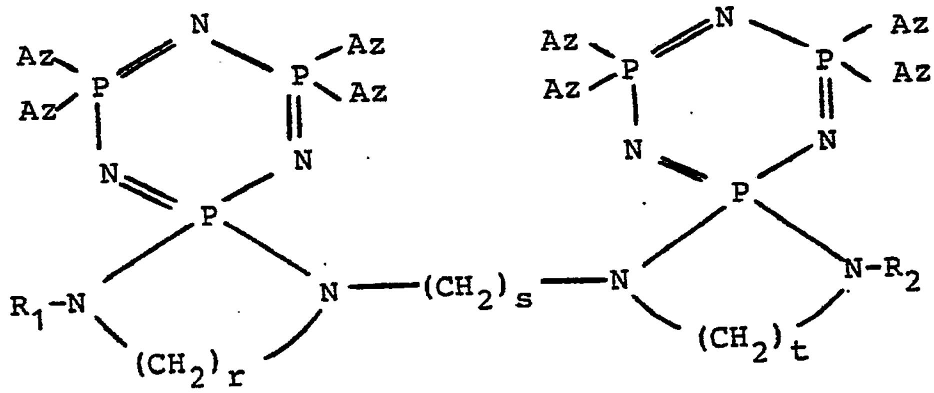

(I')

r, s et t représentant chacun, indépendamment l'un de l'autre, un nombre entier compris entre 1 et 6.

Parmi les composés de l'invention, il faut citer le composé dénommé SPM dans lequel Az = 1-aziridinyle, $R_1 = R_2 = H$, r = t = 3 et s = 4.

La présente invention concern également des procédés de préparation des composés de formule I dans lesquels :

a) on fait réagir la polyamine substituée ou non de formule II :
$H(R_1N)\text{-}A\text{-}NH\text{-}B\text{-}NH\text{-}C\text{-}(NR_2)H$ (II)
sur le composé gem dichloré de formule III en excès :

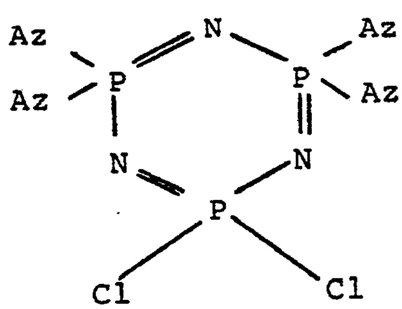

(III)

ou bien

b) on fait réagir l'aziridine substituée ou non en excès sur le composé de formule IV :

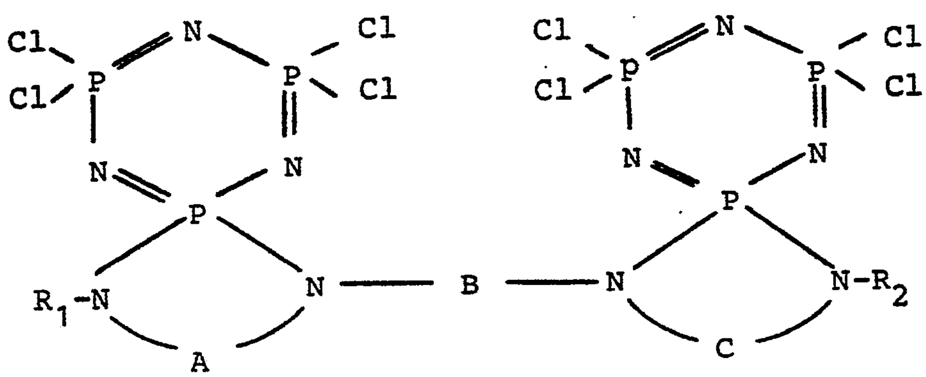

(IV)

Les composés de formule III et IV peuvent être préparés à partir de l'hexachlorocyclotriphosphazène $N_3P_3Cl_6$ de formule V :

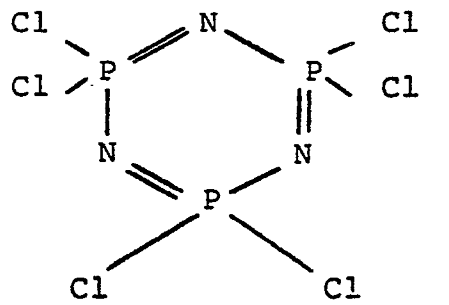

(V)

. soit en faisant réagir l'aziridine, substituée ou non, pour préparer le composé de formule III,
. soit en faisant réagir la polyamine, substitueé ou non, de formule II, pour obtenir le composé de formule IV.

Ces différentes réactions sont effectuées, de préférence, en présence d'un agent capteur de HCl qui est, de préférence, une amine ne réagissant pas sur le noyau de base,telle qu'une trialcoylamine par exemple.

Ces différentes réactions peuvent être aussi effectuées sans agent capteur de HCl, mais en présence d'un large excès stoechiométrique du réactif utilisé en excès, à savoir le composé de formule III dans la variante a) des procédés, ou l'aziridine dans la variante b) des procédés.

Ainsi, selon la variante a) des procédés de préparation des composés de formule I, on peut, en présence d'un agent capteur de HCl, effectuer la réaction en utilisant la proportion de 2 molécules du composé gem dichloré de formule III pour 1 molécule de la polyamine de formule II.

Ce rapport de 2:1 peut passer à 4:1 ou plus, dans le cas où l'on effectue la réaction sans utiliser d'agent capteur de HCl.

De même, selon la variante b) des procédés de préparation des composés de formule I, on peut, en présence d'un agent capteur de HCl, faire réagir 8 molécules d'aziridine avec 1 molécule du composé de formule IV ; par contre, en l'absence d'agent capteur de HCl, ce rapport de 8:1 peut être augmenté jusqu'à 16:1 ou plus.

Ces réactions sont conduites, de préférence, dans un solvant tel qu'un éther ou un solvant halogéné, ou un mélange de tels solvants, par exemple un mélange d'éther de pétrole 60-80° et de dichlorométhane dans le rapport 4:1. Le tétrahydrofuranne peut être également utilisé. Les réactions se déroulent, de préférence, à une température comprise entre -20°C et +20°C, par exemple à une température voisine de 0°C, et en atmosphère inerte ($N_2$ ou argon soigneusement desséché).

Parmi les polyamines utilisables selon la présente invention, il faut citer la spermine, substituée ou non, de formule : $H_2N-(CH_2)_3-NH-(CH_2)_4-NH-(CH_2)_3-NH_2$.

Enfin, le produit de la réaction de formule I peut être purifié par des procédés connus tels que l'extraction par solvant, la cristallisation, la chromatographie en phase liquide à haute performance par exemple.

Les différents produits de départ utiles dans la mise en oeuvre des procédés selon la présente invention peuvent être préparés, notamment, par les méthodes décrites dans :
-Y. KOBAYASHI, L.A. CHASIN et L.B. CLAPP, Inorg. Chem., 2, 212 (1963),
- R. RATZ, E. KOBER, C. GRUNDMANN et G. OTTMANN, Inorg. Chem., 3, 757 (1964) et brevet des E.U.A. n° 3 197 464, 27 juillet 1965,
-G. GUERCH, J-F. LABARRE, F. SOURNIES, M. MANFAIT, F. SPREAFICO et S. FILIPPESCHI, Inorg. Chim. Acta, 66, 175-183 (1982),
pour le MYCLAz,
et
- G. GUERCH, Thèse de Doctorat d'Etat n° 1118, Université Paul Sabatier, Toulouse, France 2 décembre 1983,
- J-F. LABARRE, G. GUERCH, F. SOURNIES, R. LAHANA, R. ENJALBERT et J. GALY, J. Mol. Struct., 116, 75 (1984),
- J-F. LABARRE, Topics in Current Chemistry, 129, 173 (1985), pour le composé IV.

Enfin, la présente invention concern, à titre de médicament nouveau, certains composés tels que décrits précédemment ainsi que des compositions pharmaceutiques comprenant à titre de principe actif au moins l'un de ces composés et, en particulier, des compositions pharmaceutiques administrables par voie parentérale.

Parmi les compositions administrables par voie parentérale, il faut citer les compositions injectables, en particulier par voie intra-péritonéale et intra-veineuse.

Les produits en cause étant, en général, assez solubles dans l'eau, on utilisera de préférence comme support pharmaceutique un solvant aqueux, par exemple une solution physiologique de chlorure de sodium. Bien entendu, dans le cas de produits difficiles à solubiliser, on peut utiliser des tampons fixant le pH ou éventuellement des solvants non aqueux tels que des esters, des alcools, des polyols ou diverses

huiles en combinaison avec des agents émulsifiants ; on peut également utiliser des suspensions, en particulier des hydroxypropylcelluloses.

Bien que les compositions pharmaceutiques selon la présente invention soient, de préférence, des compositions injectables, on peut également utiliser des compositions administrables par voie sublinguale, orale ou rectale, ces compositions pouvant se présenter sous forme solide, comprimés, capsules, granulés, gélules, suppositoires par exemple, ou sous forme liquide, gouttes et ampoules.

Dans le cas des compositions administrables par voie orale, celles-ci sont formulées avec des supports connus solides tels que gélatine, gomme arabique, lactose, amidon, polyalkylène glycol , carboxyméthylcellulose par exemple.

Dans le cas de suppositoires, on peut utiliser des polyéthylène glycol, de la lanoline à titre de véhicule.

Les compositions selon la présente invention peuvent également être applicables par voie topique, en particulier sous forme de pommade ou de gel applicables sur la peau avec un véhicule inerte tel que la vaseline, les polyéthylène glycol ou d'autres excipients gras ; il est possible également de prévoir un agent facilitant la pénétration cutanée du principe actif.

De façon générale, les compositions selon la présente invention peuvent contenir des adjuvants variés tels que conservateurs, stabilisants, mouillants, émulsifiants, agents de texture, de délitement, aromatisation, colorants.

Les composés selon la présente invention se sont révélés particulièrement actifs dans le traitement de certaines tumeurs. Ils se sont, en particulier, révélés très actifs sur les tumeurs standards que sont la leucémie P388, l'érythroleucémie humaine K562, la leucémie L1210, le carcinome du poumon de Lewis, le mélanome B16 et le mastocytome 5076.

Ces composés manifestent également des propriétés immuno-modulatrices remarquables, au niveau notamment de l'immuno-modulation de la stimulation polyclonale des lymphocytes B, de la prévention des lésions de glomérulonéphrites à complexes immuns et de la prévention du choc endotoxinique. Ces substances peuvent donc être envisagées pour le traitement de certaines affections à complexes immuns telles que les maladies auto-immunes et notamment le lupus érythémateux disséminé.

Ces composés sont utilisables essentiellement par injection, en particulier par voie intra-péritonéale ou par voie intra-veineuse. Ces composés étant très solubles dans l'eau, la réalisation de compositions injectables ne présente aucun problème. Les doses appliquées sont, bien entendu, fonction, dans chacun des cas, du type de tumeur à traiter ainsi que de l'état général du malade, et peuvent varier dans une très large mesure, en particulier entre 1 mg/kg/jour et 400 mg/kg/jour, en une ou plusieurs injections dont la fréquence peut être variable.

Les exemples et figure suivants sont destinés à illustrer la préparation et les activités de certains des composés selon la présente invention sans pour autant la limiter.

## EXEMPLE 1

Préparation du composé SPM à partir de son précurseur chloré de formule:
$[N_3P_3Cl_4[HN-(CH_2)_3-N](CH_2)_2]_2$

Une solution de 581,4 mmoles d'aziridine dans 100 ml de tétrahydrofuranne anhydre est ajoutée goutte à goutte en 2 heures à une solution de 19,95 mmoles du précurseur chloré dans 350 ml du même solvant. L'agitation magnétique est maintenue durant 48 heures sous atmosphère d'argon et sur bain de glace. On considère que la réaction est terminée quand les fréquences d'élongation IR (518 et 570 $cm^{-1}$) des liaison P-Cl du précurseur ont disparu. Le chlorhydrate d'aziridine formé est éliminé par filtration et le solvant de la solution mère est évaporé sous vide à 30°C. Le résidu ainsi obtenu contient encore une petite quantité de chlorhydrate. Il est donc lavé avec 20 ml d'eau glacée et extrait à trois reprises avec chaque fois 250 ml de dichlorométhane. On obtient ainsi, après évaporation sous vide de $CH_2Cl_2$, 11,2 g (70 %) d'une poudre blanche dont le Rf en chromatographie sur couche mince est égal à 0,46 avec $CH_3OH$ comme éluant. L'analyse élémentaire de l'échantillon obtenu est tout à fait en accord avec la structure du composé SPM attendu.

## EXEMPLE 2

Spectre de masse de SPM

Le spectre de masse enregistré sur un spectromètre R1010 Ribermag en utilisant un système d'injection directe a permis de mettre en évidence les caractéristiques suivantes :

L'ion miléculaire $M^+$ est observé comme attendu à m/z 804 avec 2 satellites à m/z 805 et 806 dus à la distribution isotopique $^{14}N/^{15}N$.

Le mode de fragmentation principal de $M^+$ donne simultanément les fragments $N_3P_3Az_4[HN-(CH_2)_3-N](CH_2)_4$ (M1, m/z 430) et $N_3P_3Az_4[HN-(CH_2)_3-N]$ (M2, m/z 374).

La perte successive par M1 de 1 à 4 $CH_2$ puis de la boucle SPIRO $[HN-(CH_2)_3-N]$ pas à pas et enfin de 1 à 4 radicaux Az donne les pics d'intensité maximale à m/z 416, 402, 388, 374, 360, 345 (pic de base I = 100 %), 332, 317, 304, 261, 220, 179 et 135.

La perte successive par M2 d'abord de sa boucle SPIRO et ensuite de 1 à 4 radicaux Az (associée ici encore avec des transferts d'hydrogène) donne les pics à m/z 360, 345, 332, 317, 304, 261, 220, 179 et 135. Un mode de fragmentation de moindre importance existe également par M2 qui passe d'abord par la perte de 1 à 4 radicaux Az pour donner les pics à m/z 332, 292, 248 et 207. Le fragment m/z 207 perd ensuite sa boucle SPIRO pas à pas pour donner les pics à m/z 194, 179, 165, 151 et 135.

Il est intéressant de signaler que le pic de base (I = 100 %) pour SPM correspond au fragment $N_3P_3Az_4$ [HN-$(CH_2)_2$] alors qu'il correspond à l'entité $N_3P_3Cl_4$ [HN-$(CH_2)_3$-N] $(CH_2)_4$ pour son précurseur chloré.

L'intensité des autres pics de la figure que ceux que nous avons assignés est inférieure à 0,5 %.

RMN$^{31}$P en solution dans $CD_2Cl_2$ (par rapport à $H_3PO_4$, 85 %).

Le spectre $^{31}$P de SPM, enregistré sur un Bruker WH90 (36,43 MHz) est un spectre $AX_2$ comportant un doublet à 38,48 et 37,59 ppm (entité $PAz_2$) et un triplet à 22,58, 21,69 et 20,88 ppm (atomes P portant les boucles SPIRO). La constante de couplage $^2J_{PP}$ est égale à 32,36 Hz.

EXEMPLE 3

Propriétés pharmacologiques du composé SPM

Le composé SPM a été testé sur le plan de son activité pharmacologique, notamment quant à ses propriétés antitumorales in vivo.

La solubilité dans l'eau et dans le sérum physiologique est supérieure à 20 g/l ce qui est donc largement suffisant pour la réalisation de formes injectables.

La toxicité de SPM a été déterminée sur des souris mâles Swiss ou CD2F1. La léthalité, quand elle existe, apparaît systématiquement aux jours 5 et 6 après l'adminstration par voie i.p. L'enregistrement de cette léthalité en fonction de la dose injectée permet de déduire la DLo qui est la dose maximale non léthale. La même valeur de DLo a été obtenue chez les Swiss et les CD2F1 : elle a été trouvée égale à 1 500 mg/kg. C'est là un résultat remarquable.

EXEMPLE 4

Activité antitumorale

Les tests d'activité antitumorale ont été conduits au sein du Screening and Pharmacology Group de l'E.O.R.T.C., implanté à l'Institut Mario Negri de Milan.

Ces essais ont été conduits soit en mono-injection à différentes doses au jour J+1 suivant la greffe de la tumeur, soit en poly-injections chroniques Q1D, soit en protocoles Q4D.

Les résultats obtenus sont rassemblés dans les tableaux ci-après.

L'activité antitumorale est mesurée par le rapport T/C %, T (en jours) étant le durée moyenne de vie des souris traitées, C (en jours) étant la durée moyenne de vie des souris témoins.

L'effet thérapeutique est considéré comme significatif quant T/C % est supérieur ou égal à 125.

Les tableaux appellent les remarques essentielles suivantes :

1°) Un effet remarquable est obtenu sur la leucémie P388 en mono-injection 400 mg/kg (T/C = 200 %), en protocole Q4D à 250 mg/kg/jour (T/C = 285 %) et en protocole chronique 9Q1D à 100 mg/kg/jour (T/C = 260 %) ;
on remarque surtout qu'un effet tout à fait significatif est obtenu en injectant en chronique sur 9 jours 25 mg/kg/jour (T/C = 166 %), c'est-à-dire le 1/60ème de la DLo.

2°) SPM inhibe d'une manière très remarquable la croissance in vitro de l'érythroleucémie humaine K562 alors que le MYCLAz est sans effet, même à dose toxique, sur cette souche. L'effet de SPM est significatif pour des doses comprises entre 100 µg/ml et 0,01 µg/ml, soit sur une plage d'amplitude au moins égale à 10 000.

L'action de SPM sur la K562, maladie où les cellules malignes sont des érythroblastes (globules rouges) matures donc sans noyau, donc sans ADN nucléaire, semble prouver que la cible primaire de cette nouvelle drogue n'est pas l'ADN nucléaire. On retrouve là, sous une forme directe, ce qui a été observé pour la drogue nue SOAz (étude in vitro sur les monocouches phospholipidiques modèles et in vivo sur Streptococcus pneumoniae) à savoir que la cible primaire de la plupart des cyclophosphazènes biologiquement actifs serait la membrane cellulaire (M-C. TROMBE, C. BEAUBESTRE, A-M. SAUTEREAU, J-F. LABARRE, G. LANEELLE et J-F. TOCANNE, Biochem. Pharmacology, 33, 2749 (1984).

3°) L'activité de SPM sur la leucémie L1210 est relativement faible, quel que soit le protocole utilisé. Ceci n'est pas étonnant car une telle faiblesse est commune à toutes les drogues cyclophosphazéniques décrites à ce jour.

4°) On note enfin une intéressante activité du SPM sur le carcinome du poumon de Lewis implanté chez des souris C57B1/6.

En conclusion, le composé SPM constitue, du point de vue antitumoral, une amélioration non négligeable par rapport au MYCLAz, bien que son index thérapeutique global soit objectivement moins élevé que l'on eut pu l'espérer.

On note, toutefois, une différence dans les bénéfices tirés ici de l'utilisation des composés selon la présente invention par rapport à ceux décrits dans le brevet français n° 82 19768 : il semble, en effet, que l'utilisation des composés selon la présente invention se traduise par un renforcement de la spécificité du site membranaire pour l'activité antitumorale, comme en témoignent les remarquables effets obtenus sur le K562, effets jamais approchés à ce jour.

Tableau 1

Effet de SPM sur la leucémie[a] L1210 chez les souris CD2F1

| Groupe expérimental | Schéma de traitement (mg/kg/i.p.) | T/C (%) |
|---|---|---|
| SPM | 400 x j 1 | 130 |
| | 300 x j 1 | 135 |
| | 200 x j 1 | 156 |
| | 100 x j 1 | 125 |
| | 50 x j 1 | 131 |
| | 300 x j 1, 5, 9 | 164 |
| | 200 x j 1, 5, 9 | 159 |
| | 100 x j 1, 5, 9 | 137 |
| | 50 x j 1, 5, 9 | 131 |
| | 25 x j 1, 5, 9 | 117 |
| | 150 x j 1–9 | 170 |
| | 100 x j 1–9 | 140 |
| | 75 x j 1–9 | 140 |
| | 50 x j 1–9 | 150 |
| | 25 x j 1–9 | 131 |
| | 12,5 x j 1–9 | 112 |

(a) = $10^5$ cellules ont été injectées i.p. au jour 0.

Tableau 2

Effet de SPM sur la leucémie P388[a] intracérébrale chez les souris CD2F1

| Site d'inoculation cellulaire | Dose x 9[b] (mg/kg/i.p.) | T/C (%) |
|---|---|---|
| i.p. | – | – |
| i.p. | 200 | 110 |
| | 150 | 135 |
| | 100 | 245 |
| i.c. | – | – |
| i.c. | 200 | 129 |
| | 150 | 123 |
| | 100 | 141 |

(a) = $10^6$ cellules ont été implantées pour les 2 sites d'inoculation.
(b) = SPM a été administré du jour 1 au jour 9.

Tableau 3

| Effet de SPM sur le carcinome du poumon[(a)] de Lewis chez les souris C57B1/6 | | | | |
|---|---|---|---|---|
| Groupe expérimental | Dose × j 1, 5, 9 (mg/kg/i.p.) | Poids moyen de la tumeur (g ± S.E.) | Métastases | |
| | | | Nombre moyen (N ± S.E.) | Poids moyen (mg ± S.E.) |
| Témoin | – | 9,53 ± 0,47 | 18 ± 3,4 | 209,6 ± 61,2 |
| SPM | 200 | 7,40 ± 0,33* | 14 ± 3,4 | 127,3 ± 43,1 |
| CPA[(b)] | 60 | 4,14 ± 0,27* | – | – |

(a) = $10^5$ cellules dans 0,1 ml ont été injectées i.m. au jour 0 et les animaux sacrifiés au jour 25.
(b) = Cyclophosphamide comme composé de référence.
* = $p < 0,01$ comparé au groupe témoin par le test de Duncan.

TABLEAU 4

EFFET DE SPM SUR LE CARCINOME (a) DU POUMON DE LEWIS CHEZ LES SOURIS C57B1/6

| Groupe expérimental | Dose x j 1,5,9 | Volume de la tumeur $(d^2 . \frac{D}{2}) cm^3 \pm$ S.E. par jour | | | | |
|---|---|---|---|---|---|---|
| | (mg/kg/i.p.) | 10 | 13 | 17 | 20 | 24 |
| témoin | - | 0,68±0,07 | 1,90±0,13 | 4,26±0,13 | 6,12±0,30 | 8,04±0,45 |
| SPM | 200 | 0,53±0,08 | 1,64±0,18 | 3,49±0,15* | 4,35±0,26* | 6,08±0,31* |
| CPA (b) | 60 | - | - | 0,57±0,16* | 1,30±0,17* | 3,03±0,25* |

(a) = $10^5$ cellules dans 0,1 ml ont été injectées i.m. au jour 0

(b) = Cyclophosphamide

* = $p < 0,01$ comparé au groupe témoin

Tableau 5

Effet de SPM sur le réticulo sarcome(a) M5076 murin chez les souris C57B1/6

| Groupe expérimental | Schéma de traitement (mg/kg/i.p.) | Poids corporel(b) (g ± S.E.) | Poids de la tumeur(b) (g ± S.E.) | Poids du foie(b) (g ± S.E.) | Poids de la rate(b) (mg ± S.E.) |
|---|---|---|---|---|---|
| témoin | – | 23,66 ± 0,49 | 5,2561 ± 0,18 | 3,0003 ± 0,13 | 378,8 ± 41,6 |
| SPM | 200 x j 1–9 (10/10) | 17,20 ± 0,48 | 0,3819 ± 0,13* | 1,1088 ± 0,01* | 117,8 ± 9,9* |
| | 150 x j 1–9 (10/10) | 17,55 ± 0,70 | 1,0795 ± 0,11* | 1,0190 ± 0,03* | 103,0 ± 6,02* |
| | 100 x j 1–9 (11/11) | 20,22 ± 0,37 | 2,1947 ± 0,07* | 1,1750 ± 0,03* | 137,9 ± 5,04* |
| | 50 x j 1–9 (10/10) | 20,05 ± 0,60 | 3,4033 ± 0,26* | 1,6089 ± 0,10* | 206,8 ± 12,0* |
| SPM | 300 x j 1, 5, 9 (10/10) | 20,20 ± 0,53 | 2,2664 ± 0,19* | 1,2654 ± 0,06* | 125,6 ± 10,8* |
| | 250 x j 1, 5, 9 (9/9) | 20,16 ± 0,47 | 2,4530 ± 0,18* | 1,2015 ± 0,04* | 144,5 ± 7,3* |
| | 200 x j 1, 5, 9 (10/10) | 21,05 ± 0,39 | 3,2003 ± 0,18* | 1,4620 ± 0,08* | 208,6 ± 8,8* |
| | 150 x j 1, 5, 9 (10/10) | 21,55 ± 0,29 | 3,5869 ± 0,18* | 1,7270 ± 0,10* | 240,7 ± 17,1* |
| Normal | | 20,80 ± 0,32 | | 1,0882 ± 0,02 | 81,1 ± 3,0 |

(a) = $5,10^5$ cellules tumorales ont été implantées i.m. au jour 0
(b) = déterminé au jour 23
* = p < 0,01 comparé au groupe témoin par le test de Duncan
Entre parenthèses = le nombre de souris vivantes/total

Tableau 6

Effet de SPM sur le réticulo sarcome(a) M5076 murin chez les souris C57B1/6

| Groupe expérimental | Schéma du traitement (mg/kg/i.p.) | Volume moyen de la tumeur ($cm^3$ ± S.E.) aux jours 14 | 21 |
|---|---|---|---|
| témoin | – | 0,99 ± 0,08 (10/10) | 3,52 ± 0,17 (10/10) |
| SPM | 200 x j 1–9 | – (0/10) | – (0/10) |
| | 150 x j 1–9 | – (0/10) | 0,72 ± 0* (1/10) |
| | 100 x j 1–9 | – (0/10) | 0,92 ± 0,03* (12/12) |
| | 50 x j 1–9 | 0,38 ± 0,01 (4/10) | 2,03 ± 0,17* (9/9) |
| SPM | 300 x j 1, 5, 9 | – (0/10) | 0,94 ± 0,09* (9/10) |
| | 250 x j 1, 5, 9 | 0,32 (1/9) | 1,02 ± 0,08* (8/9) |
| | 200 x j 1, 5, 9 | 0,36 ± 0,04 (2/10) | 2,02 ± 0,26* (10/10) |
| | 150 x j 1, 5, 9 | 0,41 ± 0,04 (4/10) | 2,23 ± 0,16* (10/10) |

(a) = $5.10^5$ cellules ont été implantées i.m. au jour 0
* = p < 0,01 comparé au groupe témoin par le test de Duncan
Entre parenthèses = le nombre de souris avec tumeur/total

Tableau 7

Effet de SPM sur mélanome[(a)] B16 chez les souris C57B1/6

| Groupe expérimental | Dose (mg/kg/i.p.) Schéma | Volume moyen de la tumeur ($cm^3 \pm$ S.E.) 13 | 17 | 20 | 24 | 27 | T/C (%) |
|---|---|---|---|---|---|---|---|
| témoin | | 1,37 ± 0,13 | 3,82 ± 0,19 | 5,85 ± 0,62 | 9,20 ± 0,84 | 9,73 ± 2,41 (3/9) | |
| SPM | 300 j 1, 5, 9 | 0,44 ± 0,06* | 1,72 ± 0,12* | 2,67 ± 0,24* | 5,36 ± 0,42* | 5,96 ± 0,22° (6/10) | 104 |
| | 200 j 1, 5, 9 | 0,52 ± 0,08* | 1,17 ± 0,14* | 1,66 ± 0,15* | 3,72 ± 0,29* | 4,98 ± 0,56 (8/10) | 120 |
| SPM | 200 j 1–9 | – | – | 0,12 ± 0,08* | 1,22 ± 0,17* | 2,06 ± 0,23 (9/9) | 138 |
| | 100 j 1–9 | – | 1,01 ± 0,15 | 1,71 ± 0,13* | 3,39 ± 0,34* | 3,75 ± 0,29 (9/10) | 103 |

* = $p < 0,01$ comparé au groupe témoin par le test de Duncan

° = $p < 0,05$ comparé au groupe témoin par le test de Duncan

(a) = $5.10^5$ cellules ont été implantées i.m. au jour 0

Entre parenthèses = le nombre de souris vivantes/total

Tableau 8

Effet de SPM sur le carcinome[(a)] mammaire lignée 16 chez les souris 3CH

| Groupe expérimental | Dose (mg/kg/i.p.) Schéma | Volume moyen de la tumeur ($cm^3 \pm$ S.E.) aux jours 9 | 12 | 15 | 19 | 22 | 26 | T/C (%) |
|---|---|---|---|---|---|---|---|---|
| témoin | | 0,86±0,1 | 2,71±0,25 | 4,92±0,26 | 7,50±0,49 | 9,03±0,12 | 10,21±1,15 (7/10) | – |
| SPM | 300 j 1, 5, 9 | – | – | – | 0,24± 0,14* | 0,45±0,28* | 2,24±0,31* (3/10) | 94 |
| | 200 j 1, 5, 9 | – | – | 0,37±0,05* | 1,70±0,24* | 2,43±0,31* | 3,71±0,46* (9/10) | 105 |
| SPM | 200 j 1–9 | | | | TOXIQUE | | | |
| | 100 j 1–9 | | | | TOXIQUE | | | |

(a) = $5.10^5$ cellules ont été implantées i.m. au jour 0

* = $p < 0,01$ comparé au groupe témoin par le test de Duncan

Entre parenthèses = nombre d'animaux vivants/total

Tableau 9

Effet de SPM sur la leucémie P388[(a)] chez les souris CD2F1

| Groupe expérimental | Schéma de traitement Dose (mg/kg/i.p.) | T/C (%) |
|---|---|---|
| SPM | 400 × j 1 | 200 |
| | 300 × j 1 | 166 |
| | 200 × j 1 | 166 |
| | 100 × j 1 | 152 |
| | 250 × j 1, 5, 9 | 285 |
| | 150 × j 1, 5, 9 | 219 |
| | 100 × j 1, 5, 9 | 190 |
| | 50 × j 1, 5, 9 | 161 |
| | 150 × j 1–9 | 195 |
| | 100 × j 1–9 | 260 |
| | 50 × j 1–9 | 176 |
| | 25 × j 1–9 | 166 |

(a) = $10^6$ cellules ont été implantées i.p. au jour 0

Tableau 10

Effet de SPM sur la leucémie L1210[(a)] chez les souris CD2F1

| Groupe expérimental | Schéma de traitement Dose (mg/kg/i.p.) | T/C (%) |
|---|---|---|
| SPM | 400 × j 1 | Δ |
| | 300 × j 1 | 158 |
| | 200 × j 1 | 147 |
| | 100 × j 1 | 135 |
| | 250 × j 1, 5, 9 | 194 |
| | 150 × j 1, 5, 9 | 176 |
| | 100 × j 1, 5, 9 | 152 |
| | 50 × j 1, 5, 9 | 123 |
| | 150 × j 1–9 | 182 |
| | 100 × j 1–9 | 170 |
| | 50 × j 1–9 | 152 |
| | 25 × j 1–9 | 141 |

(a) = $10^5$ cellules ont été injectées i.p. au jour 0
Δ = 2 souris mortes avant le 5ème jour, 1 souris au jour 6, 2 souris au jour 16 et la dernière était encore vivante après le 40ème jour

Tableau 11

Effet de SPM sur l'érythroleucémie humaine[(a)] K562 développée in vitro

| Groupe expérimental | Dose (µg/ml) | Nombre de cellules x $10^4$ après les heures 24 | 48 | 72 |
|---|---|---|---|---|
| témoins | – | 21,4 ± 0,6 | 36,5 ± 1,9 | 84,4 ± 4,6 |
| SPM | 100 | 11,6 ± 0,4 | 14,7 ± 0,1 | 15,0 ± 0,8 |
| | 50 | 13,2 ± 1,7 | 15,0 ± 1,5 | 17,4 ± 1,3 |
| | 10 | 17,4 ± 0,9 | 21,1 ± 0,2 | 26,9 ± 2,3 |
| | 5 | 17,3 ± 1,4 | 20,9 ± 2,1 | 35,5 ± 2,0 |
| | 1 | 17,8 ± 3,3 | 36,4 ± 1,8 | 50,9 ± 4,3 |
| | 0,1 | 17,8 ± 1,7 | 37,2 ± 4,5 | 60,6 ± 5,4 |
| | 0,01 | 20,7 ± 1,7 | 41,5 ± 1,6 | 60,7 ± 5,8 |

(a) = $10^5$ cellules/0,5 ml RPMI 1640 + 10% FCS

**Revendications pour les Etats contractants: AT, BE, CH, DE, FR, GB, IT, LI, LU, NL, SE**

1. Composés de formule I:

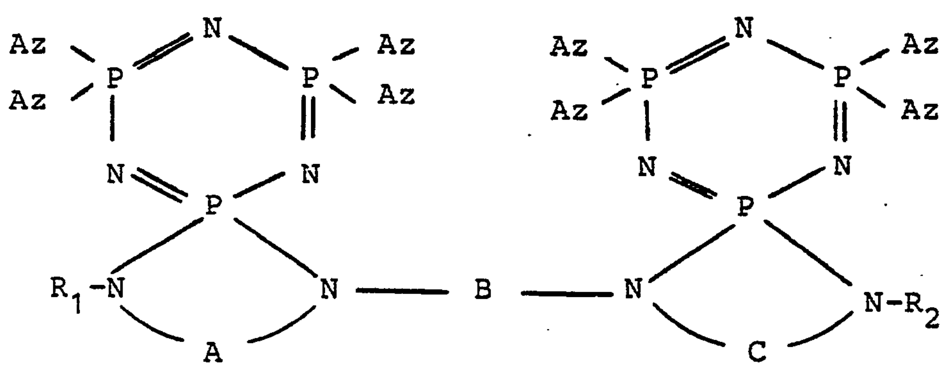

(I)

dans laquelle:

– Az est un radical 1-aziridinyle substitué ou non, la substitution étant constituée par 1 à 4 radicaux alkyles ou alkoxy en $C_1$ à $C_3$,

– A, B et C peuvent représenter, indépendamment l'un de l'autre, une chaîne alcoylène, alcénylène ou alcynylène, droite ou ramifiée, substituée ou non, qui peut être interrompue par un ou plusieurs radicaux divalents $-NR_3-$, -S- ou -O-,

– $R_1$, $R_2$ et $R_3$ peuvent représenter, indépendamment l'un de l'autre, un atome d'hydrogène, un radical alkyle substitué ou non, un radical alcényle substitué ou non, un radical alcynyle substitué ou non, un radical aryle substitué ou non,

les substitutions des chaînes -A-, -B- et -C- et des radicaux $R_1$, $R_2$ et $R_3$ étant choisies parmi un ou plusieurs atomes d'halogène, radicaux imino, amino, aryle non substitué ou substitué par un ou plusieurs atomes d'halogène ou radicaux alkyles en $C_1$ à $C_3$.

2. Composé selon la revendication 1, caractérisé en ce que Az est un radical aziridinyle non substitué.

3. Composé selon la revendication 1 de formule I':

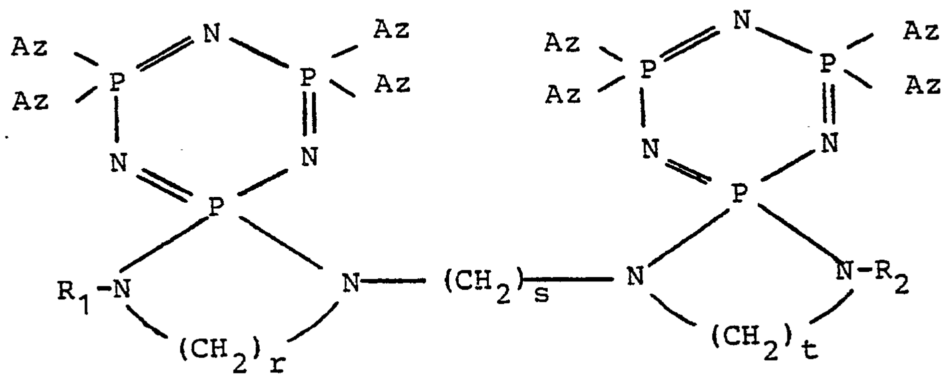

(I')

dans laquelle r, s et t représentent, indépendamment l'un de l'autre, un nombre entier compris entre 1 et 6.

4. Composé selon la revendication 3 de formule I' dans laquelle r = t = 3 et s = 4, $R_1 = R_2 = H$.

5. Procédé de préparation d'un composé selon la revendication 1, de formule I, caractérisé en ce que:

a) on fait réagir une polyamine substituée ou non de formule II:

$H(R_1N)$-A-NH-B-NH-C-$(NR_2)H$ (II)

sur le composé gem dichloré de formule III en excès:

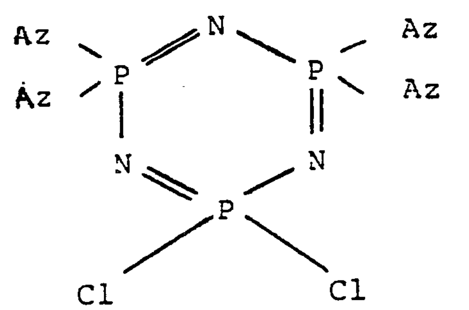

(III)

ou bien,

b) on fait réagir l'aziridine substituée ou non en excès sur le composé de formule IV:

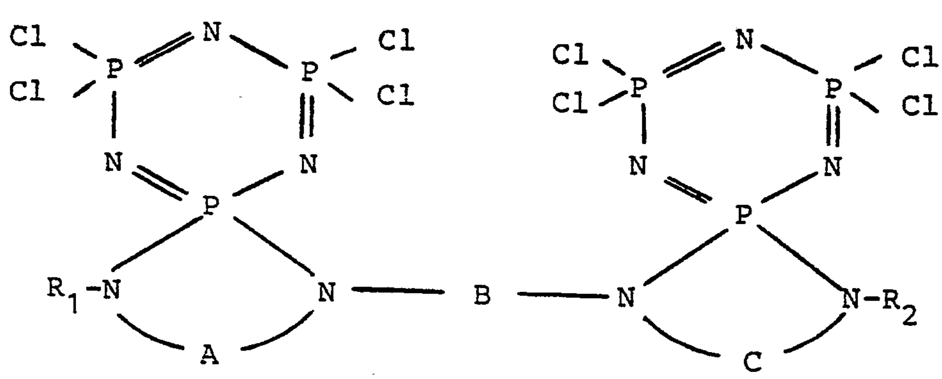

(IV)

6. Procédé selon la revendication 5, caractérisé en ce que le composé de formule III est préparé par action de l'aziridine substituée ou non sur le composé de formule V:

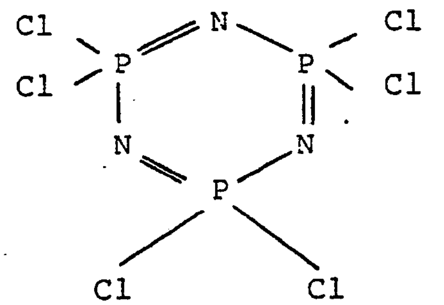

(V)

7. Procédé selon la revendication 5, caractérisé en ce que le composé de formule IV est préparé par action de la polyamine substituée ou non de formule II:
$H(R_1N)$-A-NH-B-NH-C-$(NR_2)H$ (II)
sur le composé de formule V:

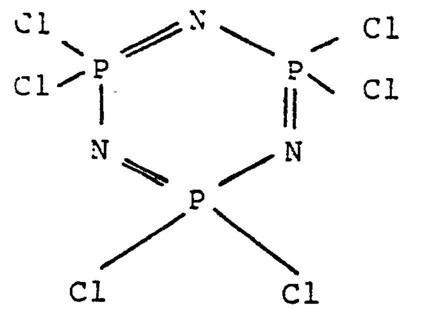

(V)

8. Procédé selon l'une des revendications 5 à 7, caractérisé en ce que la réaction est effectuée en présence d'un agent capteur de HCl.

9. Procédé selon la revendication 8, caractérisé en ce que l'agent capteur de HCl est une amine telle qu'une trialcoylamine.

10. Procédé selon l'une des revendications 5 à 7, caractérisé en ce que la réaction est effectuée en présence d'un réactif en excès.

11. Procédé selon l'une des revendications 5 à 10, caractérisé en ce que la réaction est conduite dans un solvant ou un mélange de solvants.

12. Procédé selon la revendication 11, caractérisé en ce qu'au moins un des solvants utilisés est un éther ou un solvant halogéné.

13. Procédé selon l'une des revendications 11 et 12, caractérisé en ce que la réaction est conduite dans un mélange d'éther de pétrole et de dichlorométhane.

14. Procédé selon l'une des revendications 5 à 13, caractérisé en ce que la réaction se déroule à une température comprise entre –20°C et +20°C.

15. Procédé selon l'une des revendications 5 à 14, caractérisé en ce que la réaction se déroule en atmosphère inerte.

16. Procédé selon l'une des revendications 5 à 15, caractérisé en ce que la polyamine utilisée est la spermine, substituée ou non.

17. Composition pharmaceutique comportant à titre de principe actif au moins un composé selon l'une des revendications 1 à 4.

18. Composition pharmaceutique selon la revendication 17, caractérisée en ce qu'il s'agit d'une composition injectable.

**Revendications pour les Etats contractants: ES, GR**

1. Procédé de préparation d'un composé de formule 1:

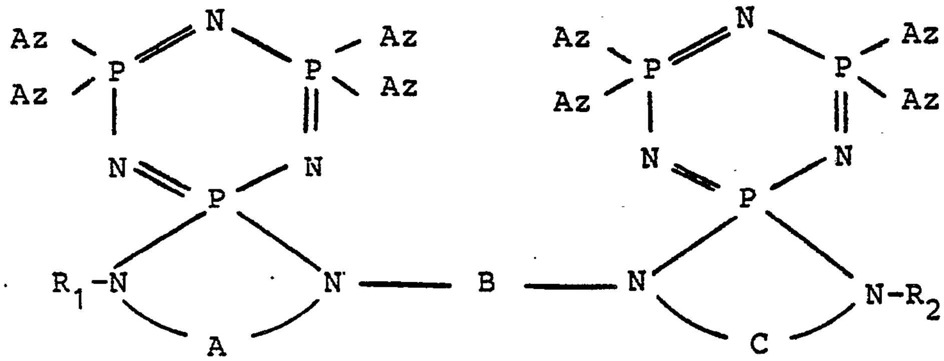

(I)

dans laquelle:
– Az est un radical 1-aziridinyle substitué ou non, la substitution étant constituée par 1 à 4 radicaux alkyles ou alkoxy en $C_1$ à $C_3$,
– A, B et C peuvent représenter, indépendamment l'un de l'autre, une chaîne alcoylène, alcénylène ou alcynylène, droite ou ramifiée, substituée ou non, qui peut être interrompue par un ou plusieurs radicaux divalents $-NR_3-$, -S- ou -O-,
– $R_1$, $R_2$ et $R_3$ peuvent représenter, indépendamment l'un de l'autre, un atome d'hydrogène, un radical alkyle substitué ou non, un radical alcényle substitué ou non, un radical alcynyle substitué ou non, un radical aryle substitué ou non,
les substitutions des chaînes -A-, -B- et -C- et des radicaux $R_1$, $R_2$ et $R_3$ étant choisies parmi un ou plusieurs atomes d'halogène, radicaux imino, amino, aryle non substitué ou substitué par un ou plusieurs atomes d'halogène ou radicaux alkyles en $C_1$ à $C_3$, caractérisé en ce que:

a) on fait réagir une polyamine substituée ou non de formule II:
$H(R_1N)-A-NH-B-NH-C-(NR_2)H$ (II)
sur le composé gem dichloré de formule III en excès:

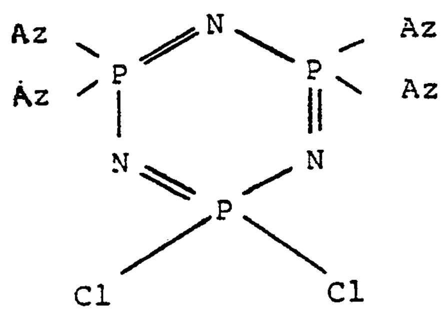

(III)

ou bien,
b) on fait réagir l'aziridine substituée ou non en excès sur le composé de formule IV:

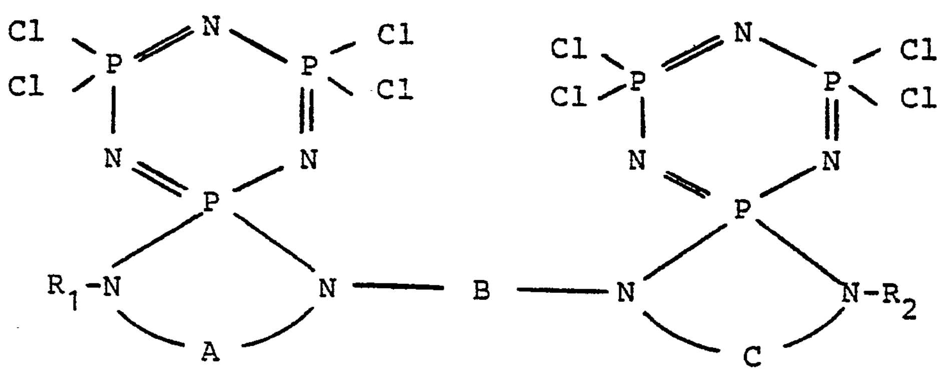

(IV)

2. Procédé selon la revendication 1, caractérisé en ce que le composé de formule III est préparé par action de l'aziridine substituée ou non sur le composé de formule V:

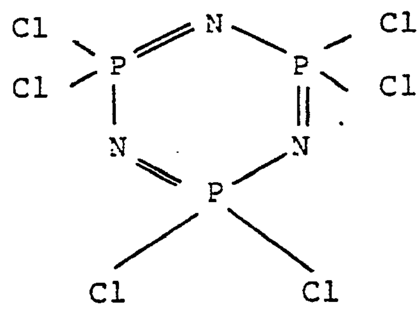

(V)

3. Procédé selon la revendication 1, caractérisé en ce que le composé de formule IV est préparé par action de la polyamine substituée ou non de formule II:
$H(R_1N)$-A-NH-B-NH-C-$(NR_2)H$ (II)
sur le composé de formule V:

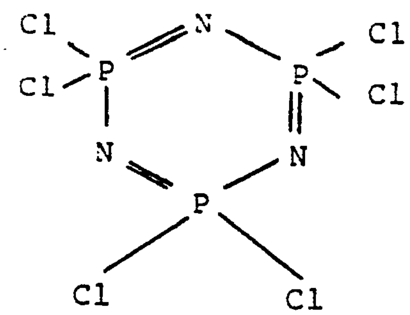

(V)

4. Procédé selon l'une des revendications 1 à 3, caractérisé en ce que la réaction est effectuée en présence d'un agent capteur de HCl.

5. Procédé selon la revendication 4, caractérisé en ce que l'agent capteur de HCl est une amine telle qu'une trialcoylamine.

6. Procédé selon l'une des revendications 1 à 3, caractérisé en ce que la réaction est effectuée en présence d'un réactif en excès.

7. Procédé selon l'une des revendications 1 à 6, caractérisé en ce que la réaction est conduite dans un solvant ou un mélange de solvants.

8. Procédé selon la revendication 7, caractérisé en ce qu'au moins un des solvants utilisés est un éther ou un solvant halogéné.

9. Procédé selon l'une des revendications 7 et 8, caractérisé en ce que la réaction est conduite dans un mélange d'éther de pétrole et de dichlorométhane.

10. Procédé selon l'une des revendications 1 à 9, caractérisé en ce que la réaction se déroule à une température comprise entre –20°C et +20°C.

11. Procédé selon l'une des revendications 1 à 10, caractérisé en ce que la réaction se déroule en atmosphère inerte.

12. Procédé selon l'une des revendications 5 à 11, caractérisé en ce que la polyamine utilisée est la spermine, substituée ou non.

**Claims for the Contracting States: AT, BE, CH, DE, FR, GB, IT, LI, LU, NL, SE**

1. Compounds having the formula I:

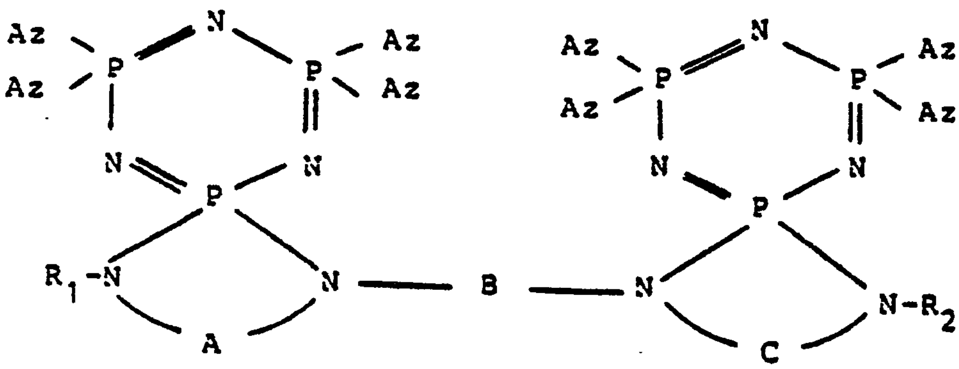

(I)

in which:
Az is a 1-aziridinyl radical, substituted or non-substituted, the substitution consisting of 1 to 4 $C_1$ to $C_3$ alkyl or alkoxy radicals,
A, B and C, independently of one another, can represent an alkylene or alkenylene or alkynylene chain which may be straight or branched, substituted or non-substituted, and can be interrupted by one or more divalent radicals $-NR_3-$, -S- or -O-, and
$R_1$, $R_2$ and $R_3$, independently of one another, can represent a hydrogen atom or a substituted or non-substituted alkyl radical or a substituted or non-substituted alkenyl radical or a substituted or non-substituted alkynyl radical or a substituted or non-substituted aryl radical,
and the substitutions of the -A-, -B- and -C- chains and the radicals $R_1$, $R_2$ and $R_3$ are chosen from among one or more halogen atoms or imino, amino or aryl radicals, non-substituted or substituted by one or more halogen atoms or $C_1$ to $C_3$ alkyl radicals.

2. A compound according to claim 1, characterised in that Az is a non-substituted aziridinyl radical.

3. A compound according to claim 1 and having the formula I':

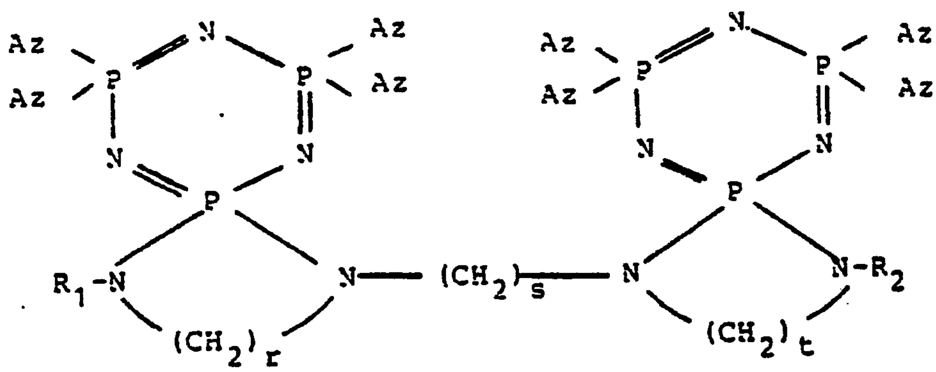

(I')

in which r, s and t, independently of one another, represent an integer between 1 and 6.

4. A compound according to claim 3 and having the formula I', in which r=t=3 and s=4, $R_1=R_2=H$.

5. A method of preparing a compound according to claim 1 and having the formula I, characterised in that

a) a substituted or non-substituted polyamine having the formula II:
$H(R_1N)-A-NH-B-NH-C(NR_2)H$ (II)
is reacted with an excess of the dichlorinated gem compound having the formula III:

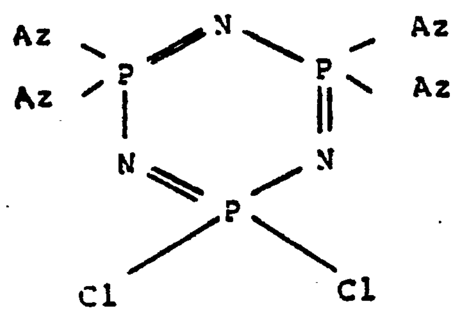

(III)

or

b) an excess of aziridine, substituted or non-substituted, is reacted with the compound having the formula IV:

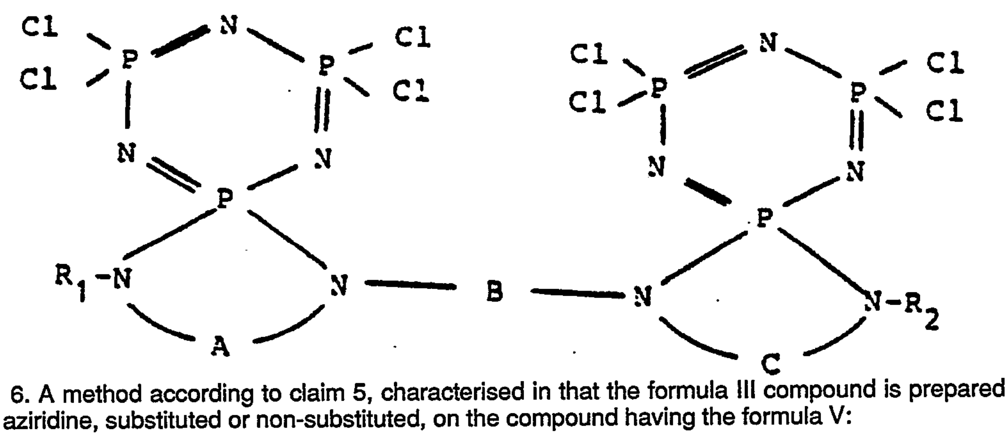

(IV)

6. A method according to claim 5, characterised in that the formula III compound is prepared by action of aziridine, substituted or non-substituted, on the compound having the formula V:

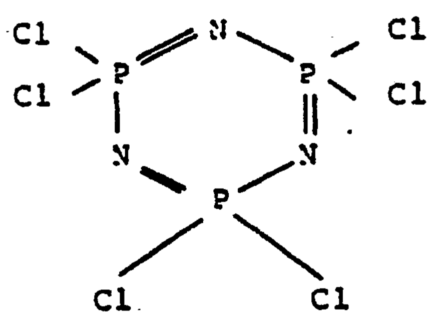

(V)

7. A method according to claim 5, characterised in that the formula IV compound is prepared by action of the substituted or non-substituted polyamine of formula II:
$H(R_1N)\text{-}A\text{-}NH\text{-}B\text{-}NH\text{-}C\text{-}(NR_2)H$ (II)
on the compound having the formula V:

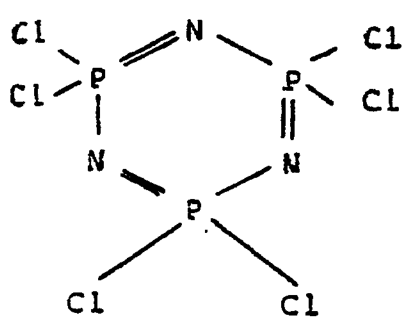

(V)

8. A method according to any of claims 5 to 7, characterised in that the reaction is brought about in the presence of an HCl-capturing agent.

9. A method according to claim 8, characterised in that the HCl-capturing agent is an amine such as trialkylamine.

10. A method according to any of claims 5 to 7, characterised in that the reaction is brought about in the presence of one reactant in excess.

11. A method according to any of claims 5 to 10, characterised in that the reaction is brought about in a solvent or a mixture of solvents.

12. A method according to claim 11, characterised in that at least one of the solvents used is an ether or halogenated solvent.

13. A method according to one of claims 11 and 12, characterised in that the reaction is brought about in a mixture of petroleum ether and dichloromethane.

14. A method according to any of claims 5 to 13, characterised in that the reaction is brought about at a temperature between –20°C and +20°C.

15. A method according to any of claims 5 to 14, characterised in that the reaction is brought about in an inert atmosphere.

16. A method according to any of claims 5 to 15, characterised in that the polyamine used is substituted or non-substituted spermine.

17. A pharmaceutical composition in which the active principle is at least one compound according to any of claims 1 to 4.

18. A pharmaceutical composition according to claim 17, characterised in that it is an injectable composition.

**Claims for the Contracting States: ES, GR**

1. A method of preparing a compound having the formula I:

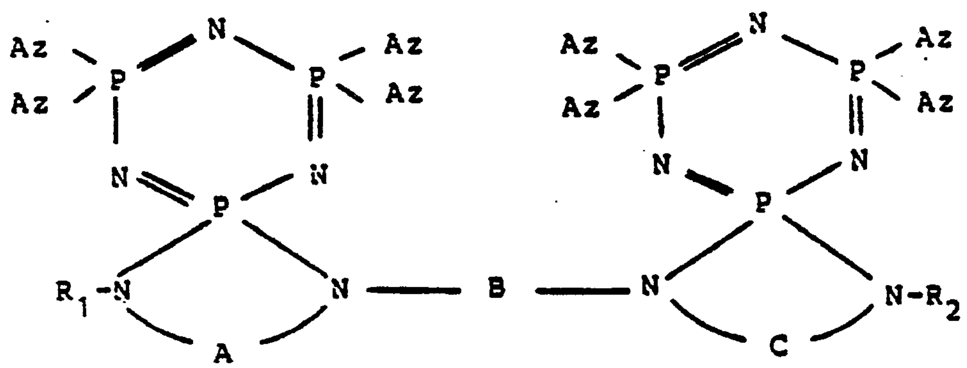

(I)

in which:

Az is a 1-aziridinyl radical, substituted or non-substituted, the substitution consisting of 1 to 4 $C_1$ to $C_3$ alkyl or alkoxy radicals,

A, B and C, independently of one another, can represent an alkylene or alkenylene or alkynylene chain which may be straight or branched, substituted or non-substituted, and can be interrupted by one or more divalent radicals $-NR_3-$, -S- or -O-, and

$R_1$, $R_2$ and $R_3$, independently of one another, can represent a hydrogen atom or a substituted or non-substituted alkyl radical or a substituted or non-substituted alkenyl radical or a substituted or non-substituted alkynyl radical or a substituted or non-substituted aryl radical,

and the substitutions of the -A-, -B- and -C- chains and the radicals $R_1$, $R_2$ and $R_3$ are chosen from among one or more halogen atoms or imino, amino or aryl radicals, non-substituted or substituted by one or more halogen atoms or $C_1$ to $C_3$ alkyl radicals, characterised in that:

a) a substituted or non-substituted polyamine having the formula II:

$H(R_1N)-A-NH-B-NH-C-(NR_2)H$ (II)

is reacted with an excess of the dichlorinated gem compound having the formula III:

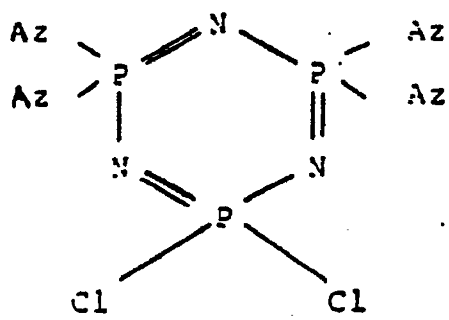

(III)

or

b) an excess of the aziridine, substituted or non-substituted, is reacted with the compound having the formula IV:

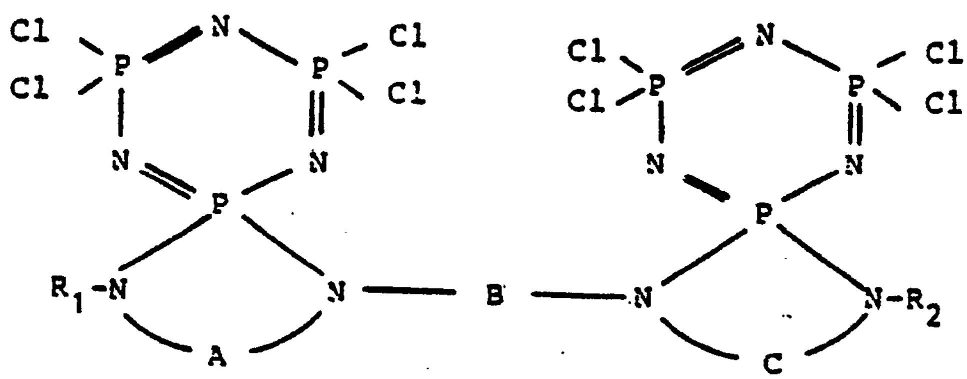

(IV)

2. A method according to claim 1, characterised in that the formula III compound is prepared by action of aziridine, substituted or non-substituted, on the compound having the formula V:

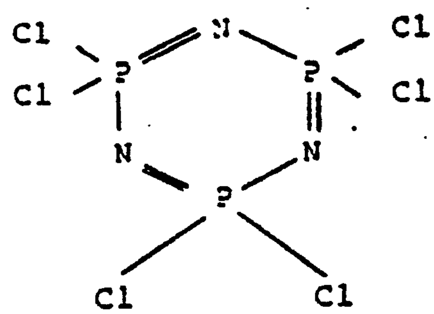

(V)

3. A method according to claim 1, characterised in that the formula IV compound is prepared by action of the substituted or non-substituted polyamine of formula II:

$H(R_1N)$-A-NH-B-NH-C-$(NR_2)H$ (II)

on the compound having the formula V:

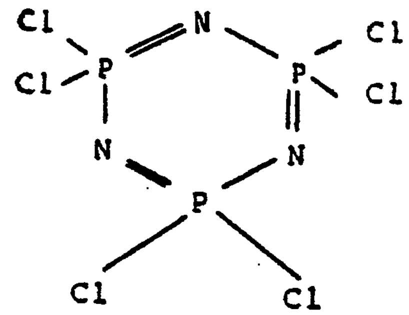

(V)

4. A method according to any of claims 1 to 3, characterised in that the reaction is brought about in the presence of an HCl-capturing agent.

5. A method according to claim 4, characterised in that the HCl-capturing agent is an amine such as trialkylamine.

6. A method according to any of claims 1 to 3, characterised in that the reaction is brought about in the presence of one reactant in excess.

7. A method according to any of claims 1 to 6, characterised in that the reaction is brought about in a solvent or a mixture of solvents.

8. A method according to claim 7, characterised in that at least one of the solvents used is an ether or a halogenated solvent.

9. A method according to claim 7 or 8, characterised in that the reaction is brought about in a mixture of petroleum ether and dichloromethane.

10. A method according to any of claims 1 to 9, characterised in that the reaction is brought about at a temperature between –20°C and +20°C.

11. A method according to any of claims 1 to 10, characterised in that the reaction is brought about in an inert atmosphere.

12. A method according to any of claims 5 to 11, characterised in that the polyamine used is substituted or non-substituted spermine.

**Patentansprüche für die Vertragsstaaten: AT, BE, CH, DE, FR, GB, IT, LI, LU, NL, SE**

1. Verbindung der Formel I:

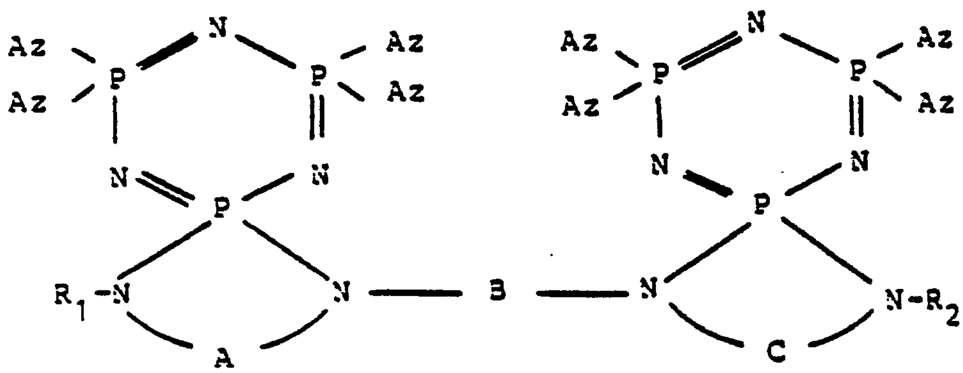

(I)

worin:

– Az ein substituierter oder nicht-substituierter 1-Aziridinylrest ist, wobei die Substituenten durch 1 – 4 Alkyl- oder Alkoxyreste mit $C_1 - C_3$ gebildet werden,

– A, B und C unabhängig voneinander eine substituierte oder nicht-substituierte, gerade oder verzweigte Alkylen-, Alkenylen- oder Alkinylenkette, die durch ein oder mehrere zweiwertige Reste $-NR_3-$, -S- oder -O- unterbrochen sein kann, darstellen können,

– $R_1$, $R_2$ und $R_3$ unabhängig voneinander ein Wasserstoffatom, einen substituierten oder nicht-substituierten Alkylrest, einen substituierten oder nicht-substituierten Alkenylrest, einen substituierten oder nicht-substituierten Alkinylrest, einen substituierten oder nicht-substituierten Arylrest darstellen können,

wobei die Substituenten der Ketten -A-, -B- und -C- und der Reste $R_1$, $R_2$ und $R_3$ aus einem oder mehreren Halogenatomen, Imino-, Amino-, Arylresten nicht substituiert oder substituiert durch ein oder mehrere Halogenatome oder Alkylreste mit $C_1 - C_3$, ausgewählt sind.

2. Verbindung nach Anspruch 1, dadurch gekennzeichnet, daß Az ein nicht-substituierter Aziridinylrest ist.

3. Verbindung nach Anspruch 1 mit der Formel I':

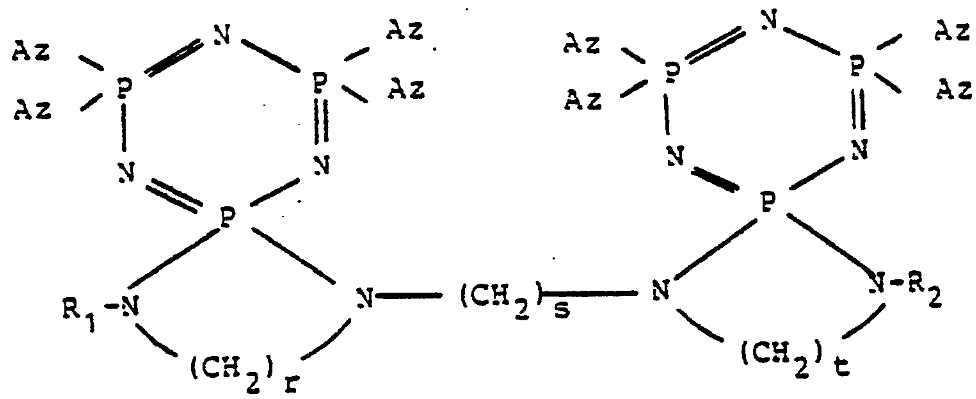

worin r, s und t unabhängig voneinander eine ganze Zahl von 1 – 6 darstellen.

4. Verbindung nach Anspruch 3, mit der Formel I', worin r=t=3 und s=4, $R_1 = R_2 = H$.

5. Verfahren zur Herstellung einer Verbindung nach Anspruch 1, mit der Formel I, dadurch gekennzeichnet, daß man:

a) ein substituiertes oder nicht-substituiertes Polyamin der Formel II

$H(R_1N)\text{-}A\text{-}NH\text{-}B\text{-}NH\text{-}C\text{-}(NR_2)H$ (II)

mit einer gem-dichlorierten Verbindung der Formel III im Überschuß reagieren läßt:

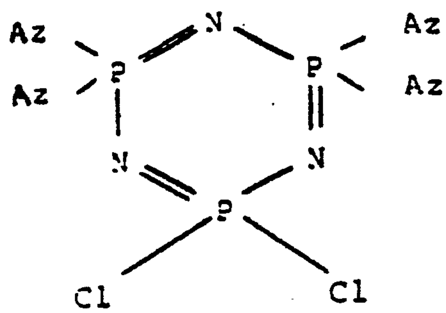

(III)

oder

b) ein substituiertes oder nicht-substituiertes Aziridin im Überschuß mit der Verbindung der Formel IV reagieren läßt:

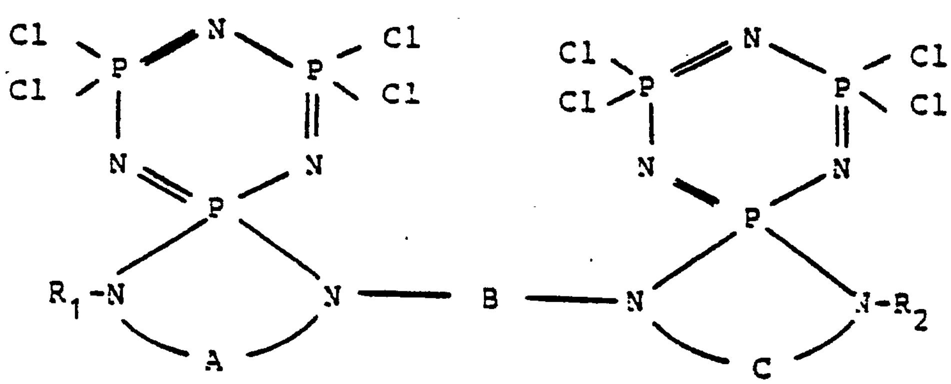

(IV)

6. Verfahren nach Anspruch 5, dadurch gekennzeichnet, daß die Verbindung der Formel III hergestellt wird durch Einwirken von substituiertem oder nicht-substituiertem Aziridin auf die Verbindung der Formel V:

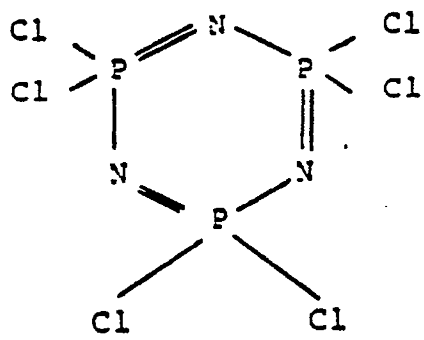

(Y)

7. Verfahren nach Anspruch 5, dadurch gekennzeichnet, daß die Verbindung der Formel IV hergestellt wird durch Einwirken von substituiertem oder nicht-substituiertem Polyamin der Formel II:
$H(R_1N)$-A-NH-B-NH-C-$(NR_2)H$ (II)
auf die Verbindung der Formel V:

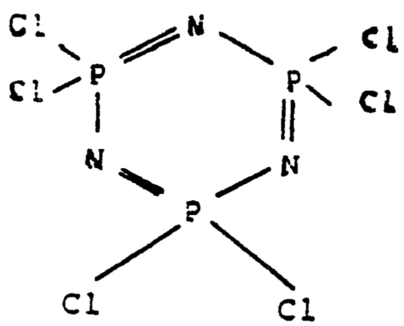

(V)

8. Verfahren nach einem der Ansprüche 5 bis 7, dadurch gekennzeichnet, daß die Reaktion in Anwesenheit eines HCl abfangenden Mittels durchgeführt wird.

9. Verfahren nach Anspruch 8, dadurch gekennzeichnet, daß das HCl abfangende Mittel ein Amin, wie ein Trialkylamin, ist.

10. Verfahren nach einem der Ansprüche 5 bis 7, dadurch gekennzeichnet, daß die Reaktion in Anwesenheit von einem Reagenz im Überschuß durchgeführt wird.

11. Vefahren nach einem der Ansprüche 5 bis 10, dadurch gekennzeichnet, daß die Reaktion in einem Lösungsmittel oder einem Lösungsmittelgemisch durchgeführt wird.

12. Verfahren nach Anspruch 11, dadurch gekennzeichnet, daß mindestens eines der verwendeten Lösungsmittel ein Ether oder ein halogeniertes Lösungsmittel ist.

13. Verfahren nach einem der Ansprüche 11 und 12, dadurch gekennzeichnet, daß die Reaktion in einem Gemisch von Petrolether und Dichlormethan durchgeführt wird.

14. Verfahren nach einem der Ansprüche 5 bis 13, dadurch gekennzeichnet, daß die Reaktion bei einer Temperatur von –20°C bis +20°C abläuft.

15. Verfahren nach einem der Ansprüche 5 bis 14, dadurch gekennzeichnet, daß die Reaktion in inerter Atmosphäre abläuft.

16. Verfahren nach einem der Ansprüche 5 bis 15, dadurch gekennzeichnet, daß das verwendete Polyamin substituiertes oder nicht-substituiertes Spermin ist.

17. Pharmazeutische Zusammensetzung, enthaltend als aktives Prinzip mindestens eine Verbindung nach einem der Ansprüche 1 bis 4.

18. Pharmazeutische Zusammensetzung nach Anspruch 17, dadurch gekennzeichnet, daß es sich um eine injizierbare Zusammensetzung handelt.

**Patentanspüche für die Vertragsstaaten: ES, GR**

1. Verfahren zur Herstellung einer Verbindung der Formel I:

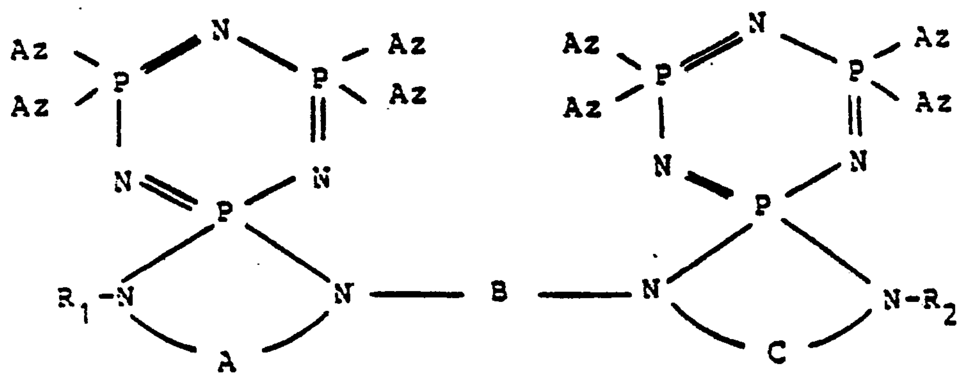

(I)

worin:

– Az ein substituierter oder nicht-substituierter 1-Aziridinylrest ist, wobei die Substituenten duch 1 – 4 Alkyl- odet Alkoxyreste mit $C_1 - C_3$ gebildet werden,

– A, B und C unabhängig voneinander eine substituierte oder nicht-substituierte, gerade oder verzweigte Alkylen-, Alkenylen- oder Alkinylenkette, die durch ein oder mehrere zweiwertige Reste -$NR_3$-, -S- oder -O- unterbrochen sein kann, darstellen können,

– $R_1$, $R_2$ und $R_3$ unabhängig voneinander ein Wasserstoffatom, einen substituierten oder nicht-substituierten Alkylrest, einen substituierten oder nicht-substituierten Alkenylrest, einen substituierten oder nicht-substituierten Alkinylrest, einen substituierten oder nicht-substituierten Arylrest darstellen können, dadurch gekennzeichnet, daß man

a) ein substituiertes oder nicht substituiertes Polyamin der Formel II

$H(R_1N)$-A-NH-B-NH-C-$(NR_2)H$ (II)

mit einer gem-dichlorierten Verbindung der Formel III im Überschuß reagieren läßt:

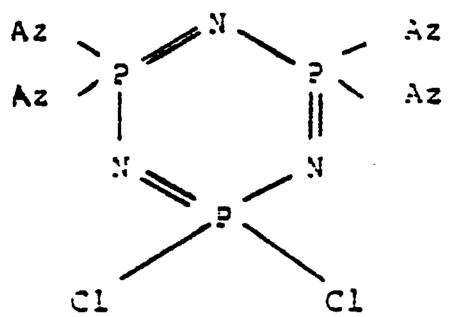

(III)

oder

b) ein substituiertes oder nicht-substituiertes Aziridin im Überschuß mit der Verbindung der Formel IV reagieren läßt:

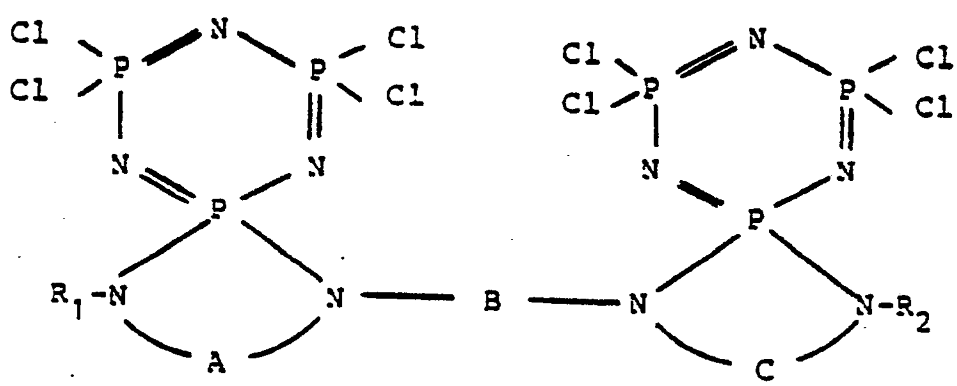

(IV)

2. Verfahren nach Anspruch 1, dadurch gekennzeichnet, daß die Verbindung der Formel III hergestellt wird durch Einwirken von substituiertem oder nicht-substituiertem Aziridin auf die Verbindung der Formel V:

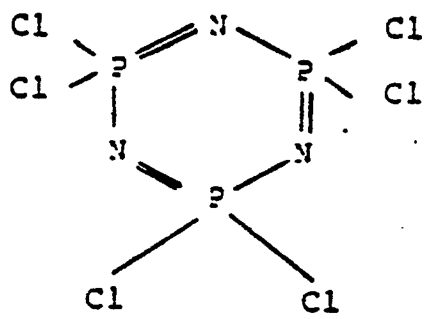

(V)

3. Verfahren nach Anspruch 1, dadurch gekennzeichnet, daß die Verbindung der Formel IV hergestellt wird durch Einwirken von substituiertem oder nicht-substituiertem Polyamin der Formel II:
$H(R_1N)$-A-NH-B-NH-C-$(NR_2)H$ (II)
auf die Verbindung der Formel V:

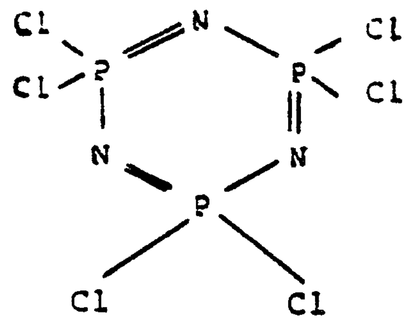

(V)

4. Verfahren nach einem der Ansprüche 1 bis 3, dadurch gekennzeichnet, daß die Reaktion in Anwesenheit eines HCl abfangenden Mittels durchgeführt wird.

5. Verfahren nach Anspruch 4, dadurch gekennzeichnet, daß das HCl abfangende Mittel ein Amin, wie ein Trialkylamin, ist.

6. Verfahren nach einem der Ansprüche 1 bis 3, dadurch gekennzeichnet, daß die Reaktion in Anwesenheit von einem Reagenz im Überschuß durchgeführt wird.

7. Verfahren nach einem der Ansprüche 1 bis 6, dadurch gekennzeichnet, daß die Reaktion in einem Lösungsmittelgemisch durchgeführt wird.

8. Verfahren nach Anspruch 7, dadurch gekennzeichnet, daß mindestens eines der verwendeten Lösungsmittel ein Ether oder ein halogeniertes Lösungsmittel ist.

9. Verfahren nach einem der Ansprüche 7 und 8, dadurch gekennzeichnet, daß die Reaktion in einem Gemisch von Petrolether und Dichlormethan durchgeführt wird.

10. Verfahren nach einem der Ansprüche 1 bis 9, dadurch gekennzeichnet, daß die Reaktion bei einer Temperatur von –20°C bis +20°C abläuft.

11. Verfahren nach einem der Ansprüche 1 bis 10, dadurch gekennzeichnet, daß die Reaktion in inerter Atmosphäre abläuft.

12. Verfahren nach einem der Ansprüche 5 bis 11, dadurch gekennzeichnet, daß das verwendete Polyamin substituiertes oder nicht-substituiertes Spermin ist.